Europäisches Patentamt

European Patent Office

Office européen des brevets

(19)

(11) Veröffentlichungsnummer : **0 170 997**
**B1**

(12) **EUROPÄISCHE PATENTSCHRIFT**

(45) Veröffentlichungstag der Patentschrift :
01.02.89

(21) Anmeldenummer : 85109393.0

(22) Anmeldetag : 26.07.85

(51) Int. Cl.⁴ : **A 61 N 1/365**, A 61 B 5/00

(54) Vorrichtung zur physiologischen Frequenzsteuerung eines mit einer Reizelektrode versehenen Herzschrittmachers.

(30) Priorität : 10.08.84 DE 3429596

(43) Veröffentlichungstag der Anmeldung :
12.02.86 Patentblatt 86/07

(45) Bekanntmachung des Hinweises auf die Patenterteilung : 01.02.89 Patentblatt 89/05

(84) Benannte Vertragsstaaten :
AT CH DE FR GB IT LI NL SE

(56) Entgegenhaltungen :
FR--A-- 2 516 797
US--A-- 4 202 339
MEDICAL AND BIOLOGICAL ENGINEERING AND COMPUTING, Band 17, Nr. 7, Juli 1979, Seiten 465-470, IFMBE, Stevenage, Herts, GB; T. SHIGEMITSU et al.: "Electrical properties of glassy-carbon electrodes"

(73) Patentinhaber : Siemens Aktiengesellschaft Berlin und München
Wittelsbacherplatz 2
D-8000 München 2 (DE)

(72) Erfinder : Mund, Konrad, Dr.
Langenbrucker Weg 10
D-8525 Uttenreuth (DE)
Erfinder : Rao, Raghavendra, Dr.
Rehweiherstrasse 29a
D-8520 Erlangen (DE)
Erfinder : Richter, Gerhard, Dr.
An der Lauseiche 30
D-8520 Erlangen (DE)

## Beschreibung

Die Erfindung betrifft eine Vorrichtung zur physiologischen Frequenzsteuerung eines mit einer Reizelektrode versehenen Herzschrittmachers mittels einer Steuereinheit sowie Verfahren zum Betrieb einer derartigen Vorrichtung.

Die gebräuchlichen Anwendungsformen der Schrittmachertherapie sind die permanente und die temporäre Elektrostimulation des Herzens. Gelegentlich wird in primär nicht eindeutig beurteilbaren Fällen die Kombination beider Verfahren erforderlich sein. Die permanente Elektrostimulation des Herzens wird angewendet bei Auftreten Adams-Stokes'scher Anfälle oder bei totalem AV-Block (AV = Atrioventrikularknoten). In Fällen von bradykarder Herzarrhythmie ist eine temporäre Elektrostimulation des Herzens vorzusehen, damit die körperliche Leistungsfähigkeit der Betroffenen verbessert wird.

Für die permanente Elektrostimulation des Herzens sind Herzschrittmacher bekannt, die einen festfrequenten Generator enthalten, der konstant beispielsweise 70 Stromstöße/Minute abgibt. Diese Herzschrittmacher sind einfach aufgebaut und wenig störanfällig, haben relativ kleine Abmessungen und erreichen auch bei normalen chemischen Batterien eine hohe Lebensdauer. Derartige Herzschrittmacher können insbesondere bei alten Menschen angewendet werden, denen ein Herzzeitvolumen auf der Basis von 70 Schlägen/Minute das noch zumutbare Ausmaß der körperlichen Belastbarkeit verschafft. Ausserdem wird ein Eigenrhythmus des Herzens des Patienten wenigstens annähernd unterdrückt. Beim Auftreten von spontanen patienteneigenen Aktionen aus einem oder mehreren Automatiezentren führt diese Parasystolie nicht nur zur irregulären Schlagfolge mit gehäuften Vorkommen gestörter Schrittmacherimpulse. Sie kann insbesondere tachykarde Zustände bis hin zum Kammerflimmern auslösen, wenn die künstlichen Stimuli in die vulnerable Phase, d. h. die T-Welle der vorangegangenen Eigenaktion, einfallen.

Außerdem sind verschiedene Typen sogenannter Bedarfsschrittmacher bekannt. Bei den Demand-Geräten wird der Impuls des Herzschrittmachers über die im Ventrikel liegende Elektrode durch das Potential der R-Zacke der Eigenaktionen solange gehemmt, wie deren Frequenz beispielsweise über 70 Schläge/Minute liegt. Sinkt sie unter diesen Wert ab, so schaltet sich das Gerät automatisch ein und übernimmt die Stimulation. Bei dem « Stand-by-Pacer » wirkt die R-Zacke des Eigenrhythmus über die Elektrode als Triggerimpuls, dem sich das Gerät im Frequenzbereich beispielsweise zwischen 70 und 150 Schläge/Minute mit angepaßter Signalaussetzung unterordnet. Fallen Eigenimpulse aus bzw. sind die R-Zackenabstände niedriger als die gerätespezifische Refraktärzeit, die beispielsweise zwischen 300 und 400 ms beträgt, wird künstlich stimuliert. Überschreiten diese indessen einen oberen vorbestimmten Minutenwert von beispielsweise 150, so halbiert der Herzschrittmacher die Frequenz, d. h. er übernimmt die elektrische Reizung des Herzen mit entsprechend reduzierter Impulsabgabe. Durch diese beiden Typen von Bedarfsschrittmachern wird die Parasystolie vermieden und man erhält ein geordnetes Nebeneinander von Eigenrhythmus und künstlicher Stimulation.

Ferner ist eine elektrochemische Vorrichtung zur Bestimmung des Sauerstoffgehaltes einer Flüssigkeit bekannt. Die Meßzelle dieser Vorrichtung besteht aus einem röhrenförmigen Körper, in dem eine Kathode und eine Anode in einem Elektrolyt angeordnet sind. Die eine Stirnseite der Meßzelle ist mit einer Membran versehen, die mit einem Dichtring und einer mit einer Öffnung versehenen Kappe befestigt ist. Diese Membran trennt die zu untersuchende Flüssigkeit von der Elektrodenanordnung. Das Meßprinzip besteht in der elektrochemischen Reduktion von Sauerstoff $(O_2)$, wobei durch die Membran ein Sauerstoffdiffusionsgrenzstrom an der Elektrode bewirkt wird. Dadurch erhält man ein konzentrationsproportionales Meßsignal (US-Patentschrift 2 913 386). Mit einer derart aufgebauten Meßzelle kann man beispielsweise die Sauerstoffkonzentration im Blut bzw. Gewebe aber nur kurzzeitig, beispielsweise für einige Tage, in vivo messen, da die Meßzelle durch sich bildende Bindegewebsschichten umschlossen und das Meßsignal somit verfälscht wird.

Aus der FR-A-2 516 797 ist ein Herzschrittmacher bekannt, dem das Meßsignal eines Sauerstoffsensors zugeführt werden kann. Dabei wird die Schrittmacherfrequenz in Abhängigkeit von der Sauerstoffkonzentration des Blutes gesteuert.

Der Erfindung liegt die Aufgabe zugrunde, einen Herzschrittmacher anzugeben, der eine der Physiologie angepaßte, einfache und wenig störanfällige Arbeitsweise ermöglicht.

Diese Aufgabe wird erfindungsgemäß gelöst durch eine Vorrichtung mit den Merkmalen des Anspruchs 1.

Bei einer ersten Ausführungsform der erfindungsgemäßen Vorrichtung wird die $O_2$-Meßelektrode jeweils parallel zur Reizelektrode mit einem Reizimpuls belastet und jeweils vor der nächsten Belastung der $O_2$-Meßelektrode (und der Reizelektrode) mit einem weiteren Reizimpuls das Potential der $O_2$-Meßelektrode, bezogen auf eine Bezugselektrode, gemessen. Das gemessene Potential entspricht der Sauerstoffkonzentration des Blutes oder des Herzmuskelgewebes. Eine Verarbeitungselektronik ordnet dabei jedem Potential der $O_2$-Meßelektrode einen Sauerstoffkonzentrationswert zu und regelt in Abhängigkeit von dieser Sauerstoffkonzentration die Frequenz, d. h. die Anzahl der Schläge/Minute des Herzens. Somit erhält man einen Herzschrittmacher mit einem implantierbaren Sauerstoffsensor, der eine der Physiologie angepaßte Arbeitsweise ermöglicht.

Bei einer zweiten Ausführungsform der erfindungsgemässen Vorrichtung wird die $O_2$-Meßelektrode ebenfalls parallel zur Reizelektrode mit einem Reizimpuls belastet. Hierbei wird jedoch jeweils vor der nächsten Belastung der $O_2$-Meßelektrode mit einem Reizimpuls die Potentialdifferenz zwischen der $O_2$-Meßelektrode und der Reizelektrode gemessen. Die $O_2$-Meßelektrode besteht vorzugsweise aus glattem Glaskohlenstoff und die Reizelektrode vorzugsweise aus aktiviertem Glaskohlenstoff. Eine derartige Reizelektrode hat eine hohe Doppelschichtkapazität, woraus eine geringe Polarisation resultiert. Bei sich schnell ändernden Sauerstoffkonzentrationen im Blut bzw. Gewebe behält die Reizelektrode aus aktiviertem Glaskohlenstoff ihr Potential bei, die $O_2$-Meßelektrode aus glattem Glaskohlenstoff ändert aber ihr Potential in Abhängigkeit von der Sauerstoffkonzentration. Durch die Differenzbildung der Potentiale werden mögliche — für beide Elektroden wirksam werdende — Einflüsse von körpereigenen Substanzen, deren Konzentrationen sich langsamer ändern als die des Sauerstoffs, eliminiert. Somit kann man sich schnell ändernde Sauerstoffkonzentrationen im Blut bzw. Gewebe messen und dementsprechend die Frequenz des Herzschrittmachers steuern.

Bei der erfindungsgemäßen Vorrichtung bilden der Herzschrittmacher und die Steuereinheit vorteilhaft eine gemeinsame Baueinheit. Außerdem können die Leitungen der $O_2$-Meßelektrode und der Bezugselektrode gemeinsam mit der Leitung der Reizelektrode in einem Elektrodenkabel angeordnet sein. Auf diese Weise erhält man einen physiologisch gesteuerten Herzschrittmacher, dessen Bauform sich gegenüber bekannten Herzschrittmacherbauformen nicht wesentlich vergrößert hat. Auch der operative Eingriff wird durch diese Bauform nicht komplizierter.

Zur weiteren Erläuterung wird auf die Zeichnung Bezug genommen, in der ein Ausführungsbeispiel einer Vorrichtung zur physiologischen Frequenzsteuerung eines mit einer Reizelektrode versehenen Herzschrittmachers schematisch veranschaulicht ist.

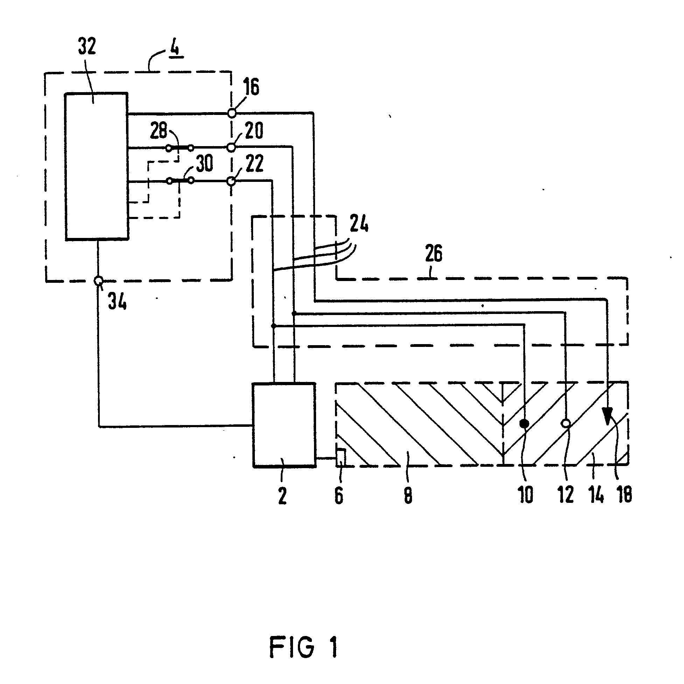

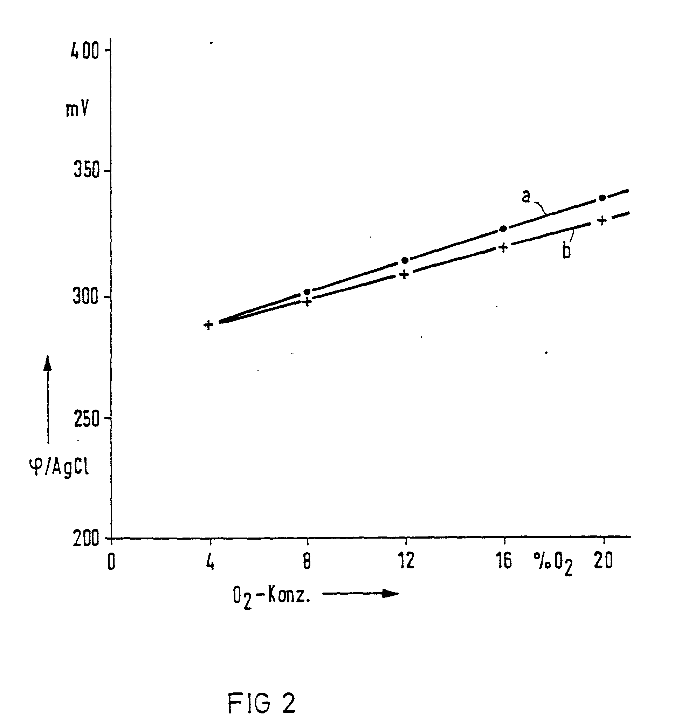

Fig. 1 zeigt eine Vorrichtung gemäß der Erfindung und in Fig. 2 sind Eichkurven des Sauerstoffsensors dargestellt.

Die in Fig. 1 dargestellte Vorrichtung enthält einen Herzschrittmacher 2 und eine Steuereinheit 4. Eine Gegenelektrode 6 des Herzschrittmachers ist mit dem Körper 8 eines Patienten verbunden. Der Herzschrittmacher 2 ist mit einer Reizelektrode 10 und einer $O_2$-Meßelektrode 12 versehen, die jeweils im Herzmuskelgewebe 14 angeordnet sind. Ein erster Eingang 16 der Steuereinheit 4 ist mit einer Bezugselektrode 18 verbunden, die in der Nähe der Reiz- bzw. $O_2$-Meßelektrode 10 bzw. 12 im Herzmuskelgewebe 14 angeordnet ist. Ein zweiter Eingang 20 der Steuereinheit 4 ist mit der $O_2$-Meßelektrode 12 und ein dritter Eingang 22 der Steuereinheit 4 mit der Reizelektrode 10 verbunden. Die $O_2$-Meßelektrode 12 besteht vorzugsweise aus glattem Glaskohlenstoff und die Reizelektrode 10 vorzugsweise aus aktiviertem Glaskohlenstoff. Die Leitungen 24 der Reiz-, $O_2$-Meß- und Bezugselektrode sind in einem gemeinsamen Elektrodenkabel angeordnet, das in der Figur durch eine gestrichelte Linie 26 dargestellt ist. Die Steuereinheit 4 enthält zwei elektronische Schalter 28 und 30 und eine Verarbeitungselektronik 32, die beispielsweise ein Mikroprozessor sein kann. Der zweite Eingang 20 und der dritte Eingang 22 der Steuereinheit 4 sind mit Hilfe der elektronischen Schalter 28 und 30 mit der Verarbeitungselektronik 32 verbunden. Die elektronischen Schalter 28 und 30 werden von der Verarbeitungselektronik 32 derart gesteuert, daß beide Schalter gleichzeitig geschlossen sind. Die Schalter 28 und 30 sind dazu durch in Fig. 1 nicht näher bezeichnete gestrichelte Linien an die Verarbeitungselektronik angeschlossen. Der erste Eingang 16 der Steuereinheit 4 ist direkt mit der Verarbeitungselektronik 32 verbunden. Der Ausgang 34 der Steuereinheit 4 ist mit dem Herzschrittmacher 2 verbunden.

Die Reizelektrode 10 und die $O_2$-Meßelektrode 12 werden parallel mit den kathodischen Reizimpulsen des Herzschrittmachers 2 belastet. Wenn der Schalter 28 geschlossen ist, wird das Potential der $O_2$-Meßelektrode 12 in bezug auf die Bezugselektrode 18 gemessen, bevor der nächste Reizimpuls die $O_2$-Meßelektrode 12 belastet. Man benutzt die Reizimpulse als Triggerimpulse. Die Zeitspanne, in der das Potential der $O_2$-Meßelektrode 12 gemessen werden kann, beträgt beispielsweise 0,5 bis 1 ms. Innerhalb dieser Zeitspanne ist das Potential der $O_2$-Meßelektrode 12 wenigstens annähernd konstant. Die Verarbeitungselektronik 32 ordnet jedem Potentialwert einen Sauerstoffkonzentrationswert zu. In Abhängigkeit von den Sauerstoffkonzentrationswerten gelangen Steuerimpulse über den Ausgang 34 der Steuereinheit 4 zum Herzschrittmacher 2. Bei ansteigender Sauerstoffkonzentration im Blut bzw. Gewebe sinkt die Anzahl der Schläge/ Minute des Herzschrittmachers 2.

Wird die Potentialdifferenz zwischen $O_2$-Meßelektrode und Reizelektrode gemessen, d. h. wird ohne zusätzliche Bezugselektrode gearbeitet, so wird auch diesen Potentialdifferenzen von der Verarbeitungselektronik jeweils ein Sauerstoffkonzentrationswert zugeordnet und somit die Anzahl der Schläge/Minute des Herzschrittmachers 2 auf einen vorbestimmten Wert erhöht bzw. erniedrigt.

In den Eichkurven nach Fig. 2 ist das Potential $\varphi$/AgCl der $O_2$-Meßelektrode 12 über der Sauerstoffkonzentration aufgetragen. Eine Gerade a mit einer positiven Steigung stellt den Potentialverlauf der $O_2$-Meßelektrode 12 in einem Elektrolyten dar, die mit kathodischen Reizimpulsen von beispielsweise 5 V und einer Impulslänge von etwa 0,5 ms belastet ist. Dieser Elektrolyt enthält beispielsweise 0,9 % Natriumchlorid (NaCl) und beispielsweise 0,1 % Natriumhydrogencarbonat ($NaHCO_3$) und bildet den Grundelektrolyt. Eine Gerade b mit positiver Steigung stellt ebenfalls den Potentialverlauf der $O_2$-Meßelektrode 12 dar. Jedoch wurden hierbei dem Grundelektrolyt physiologische Substanzen, wie beispielsweise Glucose, Harnstoff und Aminosäuremischungen in ihrer physiologisch maximalen Konzentration, zugesetzt. Die Geraden a und b beginnen jeweils im selben Ausgangspunkt, jedoch sind ihre Steigungen unterschiedlich. Die Gerade b hat beispielsweise eine Steigung von etwa 50 mV/20 % Sauerstoff. Durch die Gerade b, die nicht wesentlich von der Geraden a abweicht, wird gezeigt, daß man mit dieser Meßmethode die Sauerstoffkonzentration in vivo über einen längeren Zeitraum in Blut bzw. Gewebe auch in Anwesenheit von physiologischen Begleitsubstanzen messen kann.

**Patentansprüche**

1. Vorrichtung zur physiologischen Frequenzsteuerung eines mit einer Reizelektrode (10) versehenen Herzschrittmachers (2), dem eine $O_2$-Meßelektrode (12) zugeordnet ist, mit einer Steuereinheit (4), deren erster Eingang (16) mit einer Bezugselektrode (18), deren zweiter Eingang (20) mit der $O_2$-Meßelektrode (12), deren dritter Eingang (22) mit der Reizelektrode (10) und deren Ausgang (34) mit dem Herzschrittmacher (2) verbunden ist.

2. Vorrichtung nach Anspruch 1, dadurch gekennzeichnet, daß als Material für die $O_2$-Meßelektrode (12) glatter Glaskohlenstoff und/oder als Material für die Reizelektrode (10) aktivierter Glaskohlenstoff vorgesehen ist.

3. Vorrichtung nach Anspruch 1 oder 2, dadurch gekennzeichnet, daß die Steuereinheit (4) eine Verarbeitungselektronik (32) und wenigstens einen elektronischen Schalter (28) enthält.

4. Vorrichtung nach einem der Ansprüche 1 bis 3, dadurch gekennzeichnet, daß der zweite und dritte Eingang (20, 22) der Steuereinheit (4) über Schalter (28, 30) und der erste Eingang (16) der Steuereinheit (4) direkt mit der Verarbeitungselektronik (32) verbunden sind.

5. Vorrichtung nach Anspruch 3 oder 4, dadurch gekennzeichnet, daß ein Mikroprozessor als Verarbeitungselektronik (32) vorgesehen ist.

6. Vorrichtung nach einem oder mehreren der Ansprüche 1 bis 5, dadurch gekennzeichnet, daß die Steuereinheit (4) und der Herzschrittmacher (2) eine Baueinheit sind.

7. Verfahren zum Betrieb der Vorrichtung nach einem oder mehreren der Ansprüche 1 bis 6, dadurch gekennzeichnet, daß die $O_2$-Meßelektrode (12) parallel zur Reizelektrode (10) mit einem Reizimpuls belastet wird und daß das Potential der $O_2$-Meßelektrode (12) gegenüber der Bezugselektrode (18) gemessen wird, bevor die Reiz- und $O_2$-Meßelektrode (10, 12) wieder mit einem Reizimpuls belastet werden.

8. Verfahren zum Betrieb der Vorrichtung nach einem oder mehreren der Ansprüche 1 bis 6, dadurch gekennzeichnet, daß die $O_2$-Meßelektrode (12) parallel zur Reizelektrode (10) mit einem Reizimpuls belastet wird und daß die Potentialdifferenz zwischen $O_2$-Meßelektrode (12) und Reizelektrode (10) gemessen wird, bevor die Reiz- und $O_2$-Meßelektrode (10, 12) wieder mit einem Reizimpuls belastet werden.

9. Verfahren nach Anspruch 7 oder 8, dadurch gekennzeichnet, daß die Verarbeitungselektronik (32) gemäß dem gemessenen Potential, das ein Maß der Sauerstoffkonzentration ist, die Frequenz des Herzschrittmachers (2) ändert.

10. Verfahren nach einem der Ansprüche 7 bis 9, dadurch gekennzeichnet, daß die Schalter (28, 30) der Steuereinheit (4) von der Verarbeitungselektronik (32) derart gesteuert werden, daß beide Schalter gleichzeitig geschlossen sind.

**Claims**

1. Arrangement for the physiological frequency control of a heart pacemaker (2) which is provided with an exciting electrode (10) and with which an $O_2$-measuring electrode (12) is associated, having a control unit (4), a first input (16) of which is connected with a reference electrode (18), a second input (20) of which is connected with the $O_2$-measuring electrode (12), a third input (22) of which is connected with the exciting electrode (10) and an output (34) of which is connected with the heart pacemaker (2).

2. Arrangement according to claim 1, characterised in that smooth glassy carbon is provided as material for the $O_2$-measuring electrode (12) and/or activated glassy carbon is provided as material for the exciting electrode (10).

3. Arrangement according to claim 1 or 2, characterised in that the control unit (4) contains a processing electronic system (32) and at least one electronic switch (28).

4. Arrangement according to one of the claims 1 to 3, characterised in that the second and third input (20, 22) of the control unit (4) are connected via switches (28, 30) with the processing electronic system (32) and the first input (16) of the control unit (4) is connected directly with said processing electronic system (32).

5. Arrangement according to claim 3 or 4, characterised in that a microprocessor is provided as said processing electronic system (32).

6. Arrangement according to one or several of the claims 1 to 5, characterised in that the control unit (4) and the heart pacemaker (2) are one constructional unit.

7. Method for the operation of the arrangement according to one or several of the claims 1 to 6, characterised in that the $O_2$-measuring electrode (12) is loaded, parallel to the exciting electrode (10), with an exciting pulse and in that the potential of the $O_2$-measuring electrode (12) is measured in relation to the reference electrode (18) before the exciting and $O_2$-measuring electrodes (10, 12) are loaded again with an exciting pulse.

8. Method for the operation of the arrangement according to one or several of the claims 1 to 6, characterised in that the $O_2$-measuring electrode (12) is loaded, parallel to the exciting electrode (10), with an exciting pulse and in that the potential difference between O$_2$-measuring electrode (12) and exciting electrode (10) is measured before the exciting and O$_2$-measuring electrodes (10, 12) are loaded again with an exciting pulse.

9. Method according to claim 7 or 8, characterised in that the processing electronic system (32) varies the frequency of the heart pacemaker (2) according to the measured potential which is a measure of the oxygen concentration.

10. Method according to one of the claims 7 to 9, characterised in that the switches (28, 30) of the control unit (4) are controlled by the processing electronic system (32) such that both switches are closed at the same time.

**Revendications**

1. Dispositif pour la commande physiologique de la fréquence d'un stimulateur cardiaque (2) muni d'une électrode d'excitation (10), et auquel est coordonnée une électrode de mesure de O$_2$ (12), comprenant une unité de commande (4) dont la première entrée (16) est reliée à une électrode de référence (18), dont la deuxième entrée (20) est reliée à l'électrode de mesure de O$_2$ (12), dont la troisième entrée (22) est reliée à l'électrode d'excitation (10) et dont la sortie (34) est reliée au stimulateur cardiaque (2).

2. Dispositif selon la revendication 1, caractérisé en ce que le matériau constitutif de l'électrode de mesure de O$_2$ (12) est du carbone vitreux lisse et/ou le matériau constitutif de l'électrode d'excitation (10) est du carbone vitreux activé.

3. Dispositif selon la revendication 1 ou 2, caractérisé en ce que l'unité de commande (4) contient une électronique de traitement (32) et au moins un interrupteur électronique (28).

4. Dispositif selon une des revendications 1 à 3, caractérisé en ce que les deuxième et troisième entrées (20, 22) de l'unité de commande (4) sont reliées à travers des interrupteurs (28, 30) à l'électronique de traitement (32) et la première entrée (16) de l'unité de commande (4) est reliée directement à cette électronique de traitement.

5. Dispositif selon la revendication 3 ou 4, caractérisé en ce que l'électronique de traitement (32) est un microprocesseur.

6. Dispositif selon une ou plusieurs des revendications 1 à 5, caractérisé en ce que l'unité de commande (4) et le stimulateur cardiaque (2) forment un ensemble.

7. Procédé pour le fonctionnement du dispositif selon une ou plusieurs des revendications 1 à 6, caractérisé en ce que l'électrode de mesure O$_2$ (12) est chargée d'une impulsion d'excitation en parallèle avec l'électrode d'excitation (10) et que le potentiel de l'électrode de mesure de O$_2$ (12) est mesuré, par rapport à l'électrode de référence (18), avant qu'une nouvelle impulsion d'excitation soit appliquée aux électrodes d'excitation et de mesure de O$_2$ (10, 12).

8. Procédé pour le fonctionnement du dispositif selon une ou plusieurs des revendications 1 à 6, caractérisé en ce que l'électrode de mesure de O$_2$ (12) est chargée d'une impulsion d'excitation en parallèle avec l'électrode d'excitation (10) et que la différence de potentiel entre l'électrode de mesure de O$_2$ (12) et l'électrode d'excitation (10) est mesurée avant qu'une nouvelle impulsion d'excitation soit appliquée aux électrodes d'excitation et de mesure de O$_2$ (10, 12).

9. Procédé selon la revendication 7 ou 8, caractérisé en ce que l'électronique de traitement (32) change la fréquence du stimulateur cardiaque (2) en fonction du potentiel mesuré, lequel est une mesure de la concentration d'oxygène.

10. Procédé selon une des revendications 7 à 9, caractérisé en ce que l'électronique de traitement (32) commande les interrupteurs (28, 30) de l'unité de commande (4) de manière que les deux interrupteurs soient fermés simultanément.

FIG 1

FIG 2